(19) **Europäisches Patentamt**
**European Patent Office**
**Office européen des brevets**

(11) **EP 4 374 773 A1**

(12) **EUROPEAN PATENT APPLICATION**
published in accordance with Art. 153(4) EPC

(43) Date of publication:
**29.05.2024 Bulletin 2024/22**

(21) Application number: **22846008.5**

(22) Date of filing: **08.04.2022**

(51) International Patent Classification (IPC):
*A61B 3/117* (2006.01)     *A61B 3/00* (2006.01)
*G16H 50/20* (2018.01)     *G16H 50/30* (2018.01)
*G16H 30/40* (2018.01)     *A61B 3/135* (2006.01)
*G06N 3/08* (2023.01)      *G06N 3/04* (2023.01)

(52) Cooperative Patent Classification (CPC):
**A61B 3/00; A61B 3/117; A61B 3/135; G06N 3/04;**
**G06N 3/08; G16H 30/40; G16H 50/20; G16H 50/30**

(86) International application number:
**PCT/KR2022/005134**

(87) International publication number:
**WO 2023/003136 (26.01.2023 Gazette 2023/04)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **22.07.2021  KR 20210096560**

(71) Applicant: **Samsung Life Public Welfare**
**Foundation**
**Seoul 04348 (KR)**

(72) Inventors:
• **LIM, Dong Hui**
  **Seoul 06351 (KR)**
• **SON, Ki Young**
  **Seoul 06351 (KR)**
• **SHIN, Eun Hae**
  **Seoul 06351 (KR)**
• **KO, Jong Woo**
  **Seoul 06351 (KR)**

(74) Representative: **Weickmann & Weickmann**
**PartmbB**
**Postfach 860 820**
**81635 München (DE)**

(54) **METHOD AND SYSTEM FOR DIAGNOSIS OF CATARACT, USING DEEP LEARNING**

(57)     The present invention relates to a method and system for diagnosis of a cataract, using deep learning and, more specifically, to a method and system for diagnosis of a cataract, using deep learning, wherein deep learning is used to predict the degree of progression of a cataract from slit lamp examination result images and retroillumination examination result images and the severity of the cataract is assessed on the basis of the prediction, whereby a therapeutic plan is established and provided. A method for diagnosis of a cataract, using deep learning according to an embodiment of the present disclosure may comprise: an input step in which an input unit receives slit lamp examination result images and health diagnosis results of a subject; a cataract diagnosis step in which a cataract diagnosis unit inputs the slit lamp microscopic examination result images into a deep learning prediction model to predict a degree of progression of a cataract in the lens nucleus and determine whether the subject is affected by a cataract; a cataract severity assessment step in which a severity assessment unit assesses the severity of the cataract in the subject on the basis of the degree of progression of the cataract in the lens nucleus; and a therapeutic plan provision step in which a therapeutic step determination unit uses the severity of the cataract or the health examination result to determine and provide a necessary cataract treatment step for the subject.

EP 4 374 773 A1

【FIG. 3】

```
                    ┌─────────────────┐
                    │      START      │
                    └─────────────────┘
                             │
                             ▼
        ┌──────────────────────────────────┐
        │                                  │ ─── S100
        │         STEP OF INPUT            │
        │                                  │
        └──────────────────────────────────┘
                             │
                             ▼
        ┌──────────────────────────────────┐
        │      STEP OF DIAGNOSING          │ ─── S200
        │            CATARACT              │
        │                                  │
        └──────────────────────────────────┘
                             │
                             ▼
        ┌──────────────────────────────────┐
        │      STEP OF EVALUATING          │ ─── S300
        │      CATARACT SEVERITY           │
        │                                  │
        └──────────────────────────────────┘
                             │
                             ▼
        ┌──────────────────────────────────┐
        │      STEP OF PROVIDING           │ ─── S400
        │       TREATMENT PLAN             │
        │                                  │
        └──────────────────────────────────┘
                             │
                             ▼
                    ┌─────────────────┐
                    │       END       │
                    └─────────────────┘
```

**Description**

**[Technical Field]**

The present invention relates to a method and system for diagnosis of cataract using deep learning, and more particularly, to a method and system for diagnosis of cataract using deep learning which establishes and provides a treatment plan by estimating the degree of cataract progression from a slit lamp microscopic examination result image and a retroillumination examination result image by using the deep learning, and evaluating the cataract severity.

**[Background Art]**

Cataract refers to a disease which causes opacity of the lens, so that objects look blurred. The types of cataracts are classified into cortical, nuclear, and posterior subscapsular types depending on the location where the opacity occurs in the lens. In other words, these types are classified based on whether the location where opacity occurs in the lens is the cortex of the lens, the nucleus of the lens, or the posterior sub-capsule of the lens.

As cataract occurs, inconveniences such as decreased vision acuity or monocular diplopia, where a person sees a double image with just one eye, may arise depending on the location, degree, and extent of lens opacity.

Conventionally, as a method for diagnosing cataract, through a microscopic examination such as a slit lamp examination, a medical staff magnified and observed the lens of the patient closely to diagnose the presence, location, and progress degree of cataract disease based on subjective judgment.

However, there is a problem in that since it is a subtle task to distinguish the degrees of cataract progression, consistent diagnosis may be difficult to achieve if relying only on subjective judgment. For example, the same test may produce different diagnostic results depending on the medical staff. In other words, in order to overcome the limitations of naked eye identification, there is a need for a technology which enables automatic identification of the location, degree, and extent of lens opacity by objectifying them.

**[Summary of Invention]**

**[Technical Problem]**

In order to solve the above-mentioned problem, the present invention is to provide a method and system for diagnosis of cataract using deep learning, which can accurately analyze the degree of cataract progression of a subject by analyzing images obtained through slit lamp microscopic examination or retroillumination examination with the use of a pretrained deep learning prediction model.

**[Solution to Problem]**

As an embodiment of the present invention, a method for diagnosis of cataract using deep learning is provided.

The method for diagnosis of cataract using deep learning according to an embodiment of the present invention may include a step of input in which an input unit receives a slit lamp microscopic examination result image and a medical examination result of a subject, a step of diagnosing cataract in which a cataract diagnosing unit inputs the slit lamp microscopic examination result image into a deep learning prediction model to estimate the degree of cataract progression in a lens nucleus, and determines whether the subject has cataract, a step of evaluating cataract severity in which a severity evaluating unit evaluates the cataract severity of the subject based on the degree of cataract progression in the lens nucleus, and a step of providing treatment plan in which a treatment stage determining unit determines and provides a necessary treatment stage of cataract for the subject using the cataract severity or the medical examination result.

The method for diagnosis of cataract using deep learning according to an embodiment of the present invention may further include

before the step of input, a step of preprocessing in which a preprocessing unit extracts only a region corresponding to the lens from the slit lamp microscopic examination result image using Faster R-CNN.

In the method for diagnosis of cataract using deep learning according to an embodiment of the present invention, the degree of cataract progression in the lens nucleus may be determined by using an NO grade in which grades are classified based on the degree of opacity of the lens nucleus and an NO grade in which grades are classified based on the degree of browning of the lens nucleus.

In the method for diagnosis of cataract using deep learning according to an embodiment of the present invention, the step of diagnosing cataract may further include a step of determining that the subject has no cataract when both the NO grade and the NO grade are 0, and the step of determining that a subject has cataract when the NO grade or NO grade is not 0.

In the method for diagnosis of cataract using deep learning according to an embodiment of the present invention, the cataract severity may be divided into non-cataract, mild, moderate, and severe cataract levels, and the step of evaluating cataract severity may further include a step of evaluating the degree of cataract severity based on the greater value of the NO grade and the NO grade, and a step of extracting the visual acuity of the subject from the medical examination result and determining whether or not the visual acuity is equal to or greater than a certain standard.

In the method for diagnosis of cataract using deep learning according to an embodiment of the present invention, the step of providing treatment plan may further include in a case where the cataract severity is evaluated as non-cataract, a step of providing a schedule for a next examination when the visual acuity of the subject is equal to or greater than a certain standard, and outputting a phrase recommending a visit to a hospital when the visual acuity of the subject is less than a certain standard, in a case where the cataract severity is evaluated as mild, a step of outputting a phrase recommending a visit to a hospital, in a case where the cataract severity is evaluated as moderate, a step of outputting a phrase recommending a visit to a hospital when the visual acuity of the subject is equal to or greater than a certain standard, and outputting a phrase recommending surgery when the visual acuity of the subject is less than a certain standard, and in a case where the cataract severity is evaluated as severe, a step of outputting a phrase recommending surgery.

The method for diagnosis of cataract using deep learning according to another embodiment of the present invention may include

a step of input in which an input unit receives a retroillumination examination result image and a medical examination result of a subject, a step of diagnosing cataract in which a cataract diagnosing unit inputs the retroillumination examination result image into a deep learning prediction model to estimate the degrees of cataract progression in a cortex and a posterior subcapsular of a lens, and determines whether the subject has cataract, a step of evaluating cataract severity in which a severity evaluating unit evaluates the cataract severity of the subject based on the degrees of cataract progression in the cortex and the posterior subcapsular of the lens, and a step of providing treatment plan in which a treatment stage determining unit determines and provides a necessary treatment stage of cataract for the subject using the cataract severity or the medical examination result.

The method for diagnosis of cataract using deep learning according to an embodiment of the present invention may further include

before the step of input, a step of preprocessing in which a preprocessing unit extracts only a region corresponding to the lens from the retroillumination examination result image using Faster R-CNN.

In the method for diagnosis of cataract using deep learning according to an embodiment of the present invention, the degree of cataract progression in the cortex of lens may be determined by using a CO grade in which grades are classified based on whether opaque opacity exists in the lens cortex portion of the retroillumination examination result image and on an area occupied by the opaque opacity in the entire lens, and the degree of cataract progression in the posterior subcapsular of the lens may be determined by using a PSC grade in which grades are classified based on whether opaque opacity exists in the posterior part of the lens of the retroillumination examination result image and on an area occupied by the opaque opacity in the entire lens.

In the method for diagnosis of cataract using deep learning according to an embodiment of the present invention, the step of diagnosing cataract may further include a step of determining that the subject has no cataract when both the CO grade and the PSC grade are 0, and a step of determining that the subject has cataract when the CO grade or PSC grade is not 0.

In the method for diagnosis of cataract using deep learning according to an embodiment of the present invention, the cataract severity may be divided into non-cataract, mild, moderate, and severe cataract levels, and the step of evaluating cataract severity may further include a step of evaluating the degree of cataract severity based on the greater value of the CO grade and the PSC grade, and the step of extracting the visual acuity of the subject from the medical examination result and determining whether or not the visual acuity is equal to or higher than a certain standard.

In the method for diagnosis of cataract using deep learning according to an embodiment of the present invention, the step of providing treatment plan may further include in a case where the cataract severity is evaluated to be non-cataract, a step of providing a schedule for a next examination when the visual acuity of the subject is equal to or greater than a certain standard, and outputting a phrase recommending a visit to a hospital when the visual acuity of the subject is less than a certain standard, in a case where the cataract severity is evaluated as mild, a step of outputting a phrase recommending a visit to a hospital, in a case where the cataract severity is evaluated as moderate, a step of outputting a phrase recommending a visit to a hospital when the visual acuity of the subject is equal to or greater than a certain standard, and outputting a phrase recommending surgery when the visual acuity of the subject is less than a certain standard, and in a case where the cataract severity is evaluated as severe, a step of outputting a phrase recommending surgery.

The method for diagnosis of cataract using deep learning according to another embodiment of the present invention may include

a step of input in which an input unit receives a slit lamp microscopic examination result image, a retroillumination examination result image and a medical examination result of a subject, a step of diagnosing cataract in which a cataract diagnosing unit inputs the slit lamp microscopic examination result image, the retroillumination examination image into a deep learning prediction model to estimate the degrees of cataract progression in a nucleus, a cortex and a posterior subcapsular of a lens, and determines whether the subject has cataract, a step of evaluating cataract severity in which a severity evaluating unit evaluates the cataract severity of the subject based on the degrees of cataract progression in the nucleus, the cortex and the posterior subcapsular of the lens, and a step of providing treatment plan in which a treatment stage determining unit determines and provides a necessary treatment stage of cataract for the subject using the cataract severity or the medical examination result.

The method for diagnosis of cataract using deep learning according to an embodiment of the present invention may further include

before the step of input, a step of preprocessing in which a preprocessing unit extracts only a region corresponding to the lens from the slit lamp microscopic examination result image or the retroillumination examination result image using Faster R-CNN.

In the method for diagnosis of cataract using deep learning according to an embodiment of the present invention, the degree of cataract progression in the lens nucleus may be determined by using an NO grade in which grades are classified based on the degree of opacity of the lens nucleus and an NO grade in which grades are classified based on the degree of browning of the lens nucleus, and the degree of cataract progression in the cortex of lens may be determined by using a CO grade in which grades are classified based on whether opaque opacity exists in the lens cortex portion of the retroillumination examination result image and on an area occupied by the opaque opacity in the entire lens, and the degree of cataract progression in the posterior subcapsular of the lens may be determined by using a PSC grade in which grades are classified based on whether opaque opacity exists in the posterior part of the lens of the retroillumination examination result image and on an area occupied by the opaque opacity in the entire lens.

In the method for diagnosis of cataract using deep learning according to an embodiment of the present invention, the step of diagnosing cataract may further include a step of determining that the subject has no cataract when all of the NO grade, the NC grade, the CO grade and the PSC grade are 0, and a step of determining that the subject has cataract when the NO grade, the NC grade, the CO grade or PSC grade is not 0.

In the method for diagnosis of cataract using deep learning according to an embodiment of the present invention, the cataract severity may be divided into non-cataract, mild, moderate, and severe cataract levels, and the the step of evaluating cataract severity may further include a step of evaluating the degree of cataract severity based on the greater value of the NO grade, the NO grade, the CO grade and the PSC grade, and the step of extracting the visual acuity of the subject from the medical examination result and determining whether or not the visual acuity is equal to or greater than a certain standard.

In the method for diagnosis of cataract using deep learning according to an embodiment of the present invention, the step of providing treatment plan may further include in a case where the cataract severity is evaluated to be non-cataract, a step of providing a schedule for a next examination when the visual acuity of the subject is equal to or greater than a certain standard, and outputting a phrase recommending a visit to a hospital when the visual acuity of the subject is less than a certain standard, in a case where the cataract severity is evaluated as mild, a step of outputting a phrase recommending a visit to a hospital, in a case where the cataract severity is evaluated as moderate, a step of outputting a phrase recommending a visit to a hospital when the visual acuity of the subject is equal to or greater than a certain standard, and outputting a phrase recommending surgery when the visual acuity of the subject is less than a certain standard, and in a case where the cataract severity is evaluated as severe, a step of outputting a phrase recommending surgery.

As an embodiment of the present invention, a computer-readable recording medium on which a program for implementing the above-described method is recorded is provided.

As an embodiment of the present invention, a system for diagnosis of cataract using deep learning is provided.

A system for diagnosis of cataract using deep learning according to an embodiment of the present invention may include

an input unit obtaining a slit lamp microscopic examination result image, a retroillumination examination result image, or a medical examination result of a subject, a deep learning prediction model pretrained based on the slit lamp microscopic examination result image or the retroillumination examination result image, a cataract diagnosing unit which estimates the degree of cataract progression in the nucleus, cortex, or posterior subcapsular of a lens using the deep learning prediction model and determines whether the subject has cataract based on the degree of cataract progression, a severity evaluating unit which evaluates cataract severity based on the degree of the cataract progression, and a treatment stage determining unit which determines and provides a necessary treatment stage of cataract for the subject using the cataract severity or the medical examination result.

The system for diagnosis of cataract using deep learning according to an embodiment of the present invention may further include

a preprocessing unit which extracts only a region corresponding to the lens from the slit lamp microscopic examination result image or the retroillumination examination result image using Faster R-CNN.

In the system for diagnosis of cataract using deep learning according to an embodiment of the present invention, the preprocessing unit may increase the number of data used for the training of the deep learning prediction model by modifying the slit lamp microscopic examination result image and the retroillumination examination result image.

In the system for diagnosis of cataract using deep learning according to an embodiment of the present invention, the deep learning prediction model may be trained by extracting a fully connected layer with a fourth residual block from a pretrained network by using an ImageNet 1k data set.

In the system for diagnosis of cataract using deep learning according to an embodiment of the present invention, the deep learning prediction model may use, as an objective function, a function obtained by combining a Class Balanced (CB) loss function and a Generalized Cross Entropy (GCE) loss function.

In the system for diagnosis of cataract using deep learning according to an embodiment of the present invention, the deep learning prediction model may be used in a network with a residual block, and may be implemented by being trained on at least one network among ResNet, Wide ResNet, ResNext, and MobileNet V2, and then combining the predictions derived from the respective the networks.

[Advantageous Effects of Invention]

According to the present invention, it is possible to objectively diagnose whether a subject has cataract from a slit lamp microscopic examination result image and a retroillumination examination result image by using a pretrained deep learning prediction model.

In addition, there is a following advantage: the cataract severity of the subject can be evaluated by using the estimation result and visual acuity result, and an appropriate treatment plan can be provided depending on the degree of the severity.

Advantageous effects which can be obtained from the present invention are not limited to the aforementioned ones, and other advantageous effects not mentioned above can be clearly understood from the following detailed description by a person having ordinary skill in the art to which the disclosure belongs.

[Brief Description of Drawings]

FIG. 1(a) shows an exemplary view of a slit lamp microscopic examination result image, and FIG. 1(b) shows an exemplary view of a retroillumination examination result image.

FIG. 2 shows reference data used when a deep learning prediction model according to an embodiment of the present invention estimates the degree of cataract progression.

FIG. 3 shows a flowchart of a method for diagnosis of cataract using deep learning according to the present invention.

FIG. 4 shows a flowchart of a step of diagnosing cataract according to a first embodiment of the present invention.

FIG. 5 shows a flowchart of a step of evaluating cataract severity according to the first embodiment of the present invention.

FIG. 6 shows a flowchart of a step of diagnosing cataract according to a second embodiment of the present invention.

FIG. 7 shows a flowchart of a step of evaluating cataract severity according to the second embodiment of the present invention.

FIG. 8 shows a flowchart of a step of diagnosing cataract according to a third embodiment of the present invention.

FIG. 9 shows a flowchart of a step of evaluating cataract severity according to the third embodiment of the present invention.

FIG. 10 is a flowchart of a step of providing treatment plan according to an embodiment of the present invention.

FIG. 11 shows a block diagram of a system for diagnosis of cataract using deep learning according to an embodiment of the present invention.

[Best Mode]

As an embodiment of the present invention, a method for diagnosis of cataract using deep learning is provided.

The method for diagnosis of cataract using deep learning according to an embodiment of the present invention may include a step of input in which an input unit receives a slit lamp microscopic examination result image and a medical examination result of a subject, a step of diagnosing cataract in which a cataract diagnosing unit inputs the slit lamp microscopic examination result image into a deep learning prediction model to estimate the degree of cataract progression in a lens nucleus, and determines whether the subject has cataract, a step of evaluating cataract severity in which a severity evaluating unit evaluates the cataract severity of the subject based on the degree of cataract progression in the lens nucleus; and a step of providing treatment plan in which a treatment stage determining unit determines and provides a necessary treatment stage of cataract for the subject using the cataract severity or the medical examination result.

The method for diagnosis of cataract using deep learning according to an embodiment of the present invention may further include before the step of input, a step of preprocessing in which a preprocessing unit extracts only a region corresponding to the lens from the slit lamp microscopic examination result image using Faster R-CNN.

In the method for diagnosis of cataract using deep learning according to an embodiment of the present invention, the degree of cataract progression in the lens nucleus may be determined by using an NO grade in which grades are classified based on the degree of opacity of the lens nucleus and an NO grade in which grades are classified based on the degree of browning of the lens nucleus.

In the method for diagnosis of cataract using deep learning according to an embodiment of the present invention, the step of diagnosing cataract may further include a step of determining that the subject has no cataract when both the NO grade and the NO grade are 0, and the step of determining that a subject has cataract when the NO grade or NO grade is not 0.

In the method for diagnosis of cataract using deep learning according to an embodiment of the present invention, the cataract severity may be divided into non-cataract, mild, moderate, and severe cataract levels, and the step of evaluating cataract severity may further include a step of evaluating the degree of cataract severity based on the greater value of the NO grade and the NO grade, and a step of extracting the visual acuity of the subject from the medical examination result and determining whether or not the visual acuity is equal to or greater than a certain standard.

In the method for diagnosis of cataract using deep learning according to an embodiment of the present invention, the step of providing treatment plan may further include: in a case where the cataract severity is evaluated to be non-cataract, a step of providing a schedule for a next examination when the visual acuity of the subject is equal to or greater than a certain standard, and outputting a phrase recommending a visit to a hospital when the visual acuity of the subject is less than a certain standard; in a case where the cataract severity is evaluated as mild, a step of outputting a phrase recommending a visit to a hospital; in a case where the cataract severity is evaluated as moderate, a step of outputting a phrase recommending a visit to a hospital when the visual acuity of the subject is equal to or greater than a certain standard, and outputting a phrase recommending surgery when the visual acuity of the subject is less than a certain standard; and in a case where the cataract severity is evaluated as severe, a step of outputting a phrase recommending surgery.

The method for diagnosis of cataract using deep learning according to another embodiment of the present invention may include a step of input in which an input unit receives a retroillumination examination result image and a medical examination result of a subject, a step of diagnosing cataract in which a cataract diagnosing unit inputs the retroillumination examination result image into a deep learning prediction model to estimate the degrees of cataract progression in a cortex and a posterior subcapsular of a lens, and determines whether the subject has cataract, a step of evaluating cataract severity in which a severity evaluating unit evaluates the cataract severity of the subject based on the degrees of cataract progression in the cortex and the posterior subcapsular of the lens, and a step of providing treatment plan in which a treatment stage determining unit determines and provides a necessary treatment stage of cataract for the subject using the cataract severity or the medical examination result.

The method for diagnosis of cataract using deep learning according to an embodiment of the present invention may further include before the step of input, a step of preprocessing in which a preprocessing unit extracts only a region corresponding to the lens from the retroillumination examination result image using Faster R-CNN.

In the method for diagnosis of cataract using deep learning according to an embodiment of the present invention, the degree of cataract progression in the cortex of lens may be determined by using a CO grade in which grades are classified based on whether opaque opacity exists in the lens cortex portion of the retroillumination examination result image and on an area occupied by the opaque opacity in the entire lens, and the degree of cataract progression in the posterior subcapsular of the lens may be determined by using a PSC grade in which grades are classified based on whether opaque opacity exists in the posterior part of the lens of the retroillumination examination result image and on an area occupied by the opaque opacity in the entire lens.

In the method for diagnosis of cataract using deep learning according to an embodiment of the present invention, the step of diagnosing cataract may further include a step of determining that the subject has no cataract when both the CO grade and the PSC grade are 0, and a step of determining that the subject has cataract when the CO grade or PSC grade is not 0.

In the method for diagnosis of cataract using deep learning according to an embodiment of the present invention, the cataract severity may be divided into non-cataract, mild, moderate, and severe cataract levels, and the step of evaluating cataract severity may further include a step of evaluating the degree of cataract severity based on the greater value of the CO grade and the PSC grade, and the step of extracting the visual acuity of the subject from the medical examination result and determining whether or not the visual acuity is equal to or higher than a certain standard.

In the method for diagnosis of cataract using deep learning according to an embodiment of the present invention, the step of providing treatment plan may further include: in a case where the cataract severity is evaluated to be non-cataract, a step of providing a schedule for a next examination when the visual acuity of the subject is equal to or greater than a certain standard, and outputting a phrase recommending a visit to a hospital when the visual acuity of the subject is less than a certain standard; in a case where the cataract severity is evaluated as mild, a step of outputting a phrase recommending a visit to a hospital; in a case where the cataract severity is evaluated as moderate, a step of outputting a phrase recommending a visit to a hospital when the visual acuity of the subject is equal to or greater than a certain standard, and outputting a phrase recommending surgery when the visual acuity of the subject is less than a certain standard; and in a case where the cataract severity is evaluated as severe, a step of outputting a phrase recommending surgery.

The method for diagnosis of cataract using deep learning according to another embodiment of the present invention may include:
a step of input in which an input unit receives a slit lamp microscopic examination result image, a retroillumination examination result image and a medical examination result of a subject, a step of diagnosing cataract in which a cataract diagnosing unit inputs the slit lamp microscopic examination result image, the retroillumination examination image into a deep learning prediction model to estimate the degrees of cataract progression in a nucleus, a cortex and a posterior subcapsular of a lens, and determines whether the subject has cataract, a step of evaluating cataract severity in which a severity evaluating unit evaluates the cataract severity of the subject based on the degrees of cataract progression in the nucleus, the cortex and the posterior subcapsular of the lens, and a step of providing treatment plan in which a treatment stage determining unit determines and provides a necessary treatment stage of cataract for the subject using the cataract severity or the medical examination result.

The method for diagnosis of cataract using deep learning according to an embodiment of the present invention may further include
before the step of input, a step of preprocessing in which a preprocessing unit extracts only a region corresponding to the lens from the slit lamp microscopic examination result image or the retroillumination examination result image using Faster R-CNN.

In the method for diagnosis of cataract using deep learning according to an embodiment of the present invention, the degree of cataract progression in the lens nucleus may be determined by using an NO grade in which grades are classified based on the degree of opacity of the lens nucleus and an NO grade in which grades are classified based on the degree of browning of the lens nucleus, and the degree of cataract progression in the cortex of lens may be determined by using a CO grade in which grades are classified based on whether opaque opacity exists in the lens cortex portion of the retroillumination examination result image and on an area occupied by the opaque opacity in the entire lens, and the degree of cataract progression in the posterior subcapsular of the lens may be determined by using a PSC grade in which grades are classified based on whether opaque opacity exists in the posterior part of the lens of the retroillumination examination result image and on an area occupied by the opaque opacity in the entire lens.

In the method for diagnosis of cataract using deep learning according to an embodiment of the present invention, the step of diagnosing cataract may further include a step of determining that the subject has no cataract when all of the NO grade, the NC grade, the CO grade and the PSC grade are 0, and a step of determining that the subject has cataract when the NO grade, the NC grade, the CO grade or PSC grade is not 0.

In the method for diagnosis of cataract using deep learning according to an embodiment of the present invention, the cataract severity may be divided into non-cataract, mild, moderate, and severe cataract levels, and the the step of evaluating cataract severity may further include a step of evaluating the degree of cataract severity based on the greater value of the NO grade, the NO grade, the CO grade and the PSC grade, and the step of extracting the visual acuity of the subject from the medical examination result and determining whether or not the visual acuity is equal to or greater than a certain standard.

In the method for diagnosis of cataract using deep learning according to an embodiment of the present invention, the step of providing treatment plan may further include: in a case where the cataract severity is evaluated to be non-cataract, a step of providing a schedule for a next examination when the visual acuity of the subject is equal to or greater than a certain standard, and outputting a phrase recommending a visit to a hospital when the visual acuity of the subject is less than a certain standard; in a case where the cataract severity is evaluated as mild, a step of outputting a phrase recommending a visit to a hospital; in a case where the cataract severity is evaluated as moderate, a step of outputting a phrase recommending a visit to a hospital when the visual acuity of the subject is equal to or greater than a certain standard, and outputting a phrase recommending surgery when the visual acuity of the subject is less than a certain standard; and in a case where the cataract severity is evaluated as severe, a step of outputting a phrase recommending surgery.

As an embodiment of the present invention, a computer-readable recording medium on which a program for implementing the above-described method is recorded is provided.

As an embodiment of the present invention, a system for diagnosis of cataract using deep learning is provided.

The system for diagnosis of cataract using deep learning according to an embodiment of the present invention may include

an input unit obtaining a slit lamp microscopic examination result image, a retroillumination examination result image, or a medical examination result of a subject; a deep learning prediction model pretrained based on the slit lamp microscopic examination result image or the retroillumination examination result image; a cataract diagnosing unit which estimates the degree of cataract progression in the nucleus, cortex, or posterior subcapsular of a lens using the deep learning prediction model and determines whether the subject has cataract based on the degree of cataract progression; a severity evaluating unit which evaluates cataract severity based on the degree of the cataract progression; and a treatment stage determining unit which determines and provides a necessary treatment stage of cataract for the subject using the cataract severity or the medical examination result.

The system for diagnosis of cataract using deep learning according to an embodiment of the present invention may further include a preprocessing unit which extracts only a region corresponding to the lens from the slit lamp microscopic examination result image or the retroillumination examination result image using Faster R-CNN.

In the system for diagnosis of cataract using deep learning according to an embodiment of the present invention, the preprocessing unit may increase the number of data used for the training of the deep learning prediction model by modifying the slit lamp microscopic examination result image and the retroillumination examination result image.

In the system for diagnosis of cataract using deep learning according to an embodiment of the present invention, the deep learning prediction model may be trained by extracting a fully connected layer with a fourth residual block from a pretrained network by using an ImageNet 1k data set.

In the system for diagnosis of cataract using deep learning according to an embodiment of the present invention, the deep learning prediction model may use, as an objective function, a function obtained by combining a Class Balanced (CB) loss function and a Generalized Cross Entropy (GCE) loss function.

In the system for diagnosis of cataract using deep learning according to an embodiment of the present invention, the deep learning prediction model may be used in a network with a residual block, and may be implemented by being trained on at least one network among ResNet, Wide ResNet, ResNext, and MobileNet V2, and then combining the predictions derived from the respective the networks.

[Mode for Invention]

Hereinafter, embodiments of the present invention will be described in detail with reference to the accompanying drawings such that those of ordinary skill in the art can easily practice them. However, the disclosure can be embodied in various different forms, and the scope of the disclosure should not be construed as being limited to the embodiments described herein. In the drawings, in order to describe clearly the disclosure, parts not related to the description are omitted, and like reference signs will be given to like constitutional elements throughout the specification.

The terms used in this specification will be briefly explained, and the present invention will be described in detail.

The terms used in the present invention have been selected from among general terms currently widely used as many as possible while considering their functions in the present invention, but this may vary depending on the intentions of engineers working in the art, precedents, the emergence of new technologies, or the like. In addition, in certain cases, there are terms arbitrarily selected by the applicant, and in this case, their meanings will be described in detail in the description of the relevant disclosure. Therefore, the term used in the disclosure should be defined based on not a simple term name but the meaning of the term and the entire contents of the disclosure.

Throughout this specification, when a part "includes" or "comprises" a component, it means not that the part excludes other component, but instead that the part may further include other component unless expressly stated to the contrary. In addition, terms such as " ... unit" and "module" described in the specification mean a unit that processes at least one function or operation, which may be implemented in hardware or software, or a combination of hardware and software. Further, as used herein, "connecting" a part with another part may refer to a case where they are "connected" to each other with other element intervening therebetween, as well as a case where they are directly connected.

Hereinafter, the present invention will be described in detail with reference to the accompanying drawings.

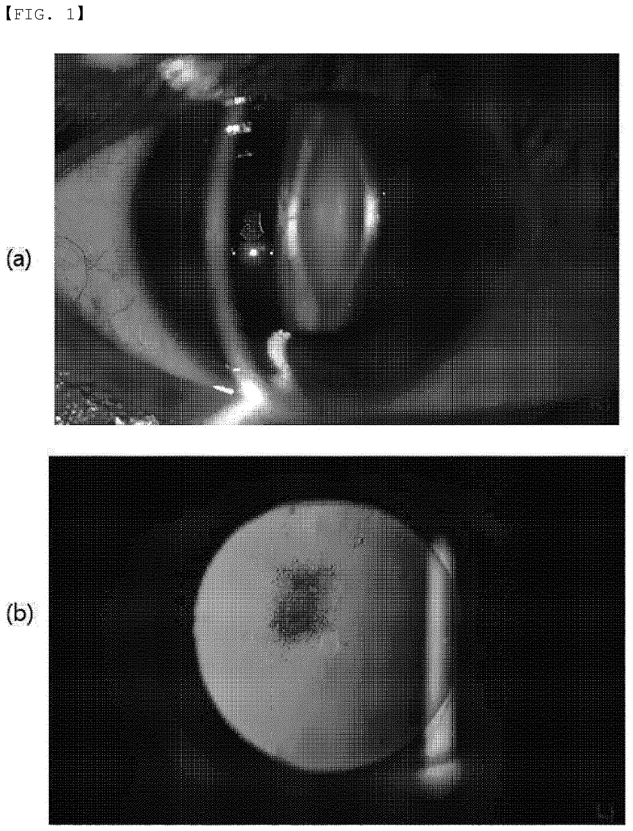

FIG. 1(a) shows an exemplary view of a slit lamp microscopic examination result image, and FIG. 1(b) shows an exemplary view of a retroillumination examination result image.

In this regard, the slit lamp examination result image refers to a magnified image of the eye of the subject taken with a slit lamp microscope equipped with a high-magnification microscope. The retroillumination examination result image refers to an image of eyeball structures such as the lens, cornea and the like observed with reflected light by illuminating light directly to the eyeball, and the retroillumination examination result image is taken in a different illumination method in the slit lamp microscopy.

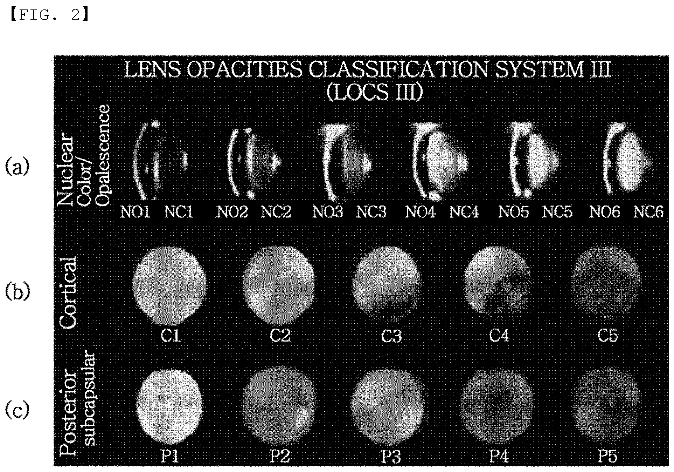

FIG. 2 shows reference data used when a deep learning prediction model 10 according to an embodiment of the present invention estimates the degree of cataract progression.

Referring to FIG. 2, the reference data may indicate the degrees of cataract progression of the subject by classifying them in terms of region of the nucleus, cortex, and posterior subcapsular of the lens.

In this regard, the degree of cataract progression in the lens nucleus may be divided into an NO grade in which grades are classified based on the degree of opacity of the lens nucleus and an NC grade in which grades are classified based on the degree of browning of the lens nucleus. In this regard, the NO grade and the NC grade may be divided into a total of 7 stages which are classified into 1 to 6 grades with 0 grade when there is no opacity or browning of the lens nucleus, as shown in FIG. 2.

In addition, the degree of cataract progression in the cortex of lens can be divided using a CO grade in which grades are classified based on whether opaque opacity exists in the lens cortex portion of the retroillumination examination result image and on the area occupied by the opaque opacity in the entire lens. In this regard, the CO grade may be divided into a total of six stages which are classified into 1 to 5 grades with a grade of 0 when there is no cortex opacity, as shown in FIG. 2.

Further, the degree of cataract progression in the posterior subcapsular region of the lens can be divided by using a PSC grade in which grades are classified based on whether opaque opacity exists in the posterior part of the lens of the retroillumination examination result image and on the area occupied by the opaque opacity in the entire lens. In this regard, the PSC grade may be divided into a total of six stages which are classified into 1 to 5 grades with a grade of 0 when there is no cortex opacity, as shown in FIG. 2.

With regard to the NO grade, NC grade, CO grade and PSC grade, a higher grade thereof means that the degree of cataract progression may be greater.

The deep learning prediction model 10 used in the method for diagnosis of cataract according to an embodiment of the present invention can be implemented as a prediction model trained by labeling the degree of cataract progression on the previously accumulated slit lamp microscopic examination result images and retroillumination examination result images.

In this case, the training means the process of estimating what grade the microscopic examination result image inputted to the deep learning prediction model corresponds to in the reference data of FIG. 2, comparing whether the estimated result matches the labeling, calculating an error rate, and then reducing the error rate.

Hereinafter, the training process of the deep learning prediction model 10 used in the method for diagnosing cataract according to an embodiment of the present invention will be described in detail.

According to an embodiment, in order to solve the overfitting problem, the deep learning prediction model 10 may increase the number of data used for the training by modifying (Data Augmentation) previously accumulated slit lamp microscopic examination result images or retroillumination examination result images.

In this case, as the method of modifying the slit lamp microscopic examination result image, method of modifying data as shown in [Table 1] below may be used.

[Table 1]

| Augmented Techniques | Hyper Parameters |
|---|---|
| Crop | RandomInt (0, 16) |
| Gaussian Blur | Normal (0.0, 3.0) |
| Flip Horizontal / Vertical | P=0.5 |
| Rotation | Uniform (-45.0, 45.0) |
| Change Brightness | Uniform (-30.0, 30.0) |
| Remove Saturation | Uniform (0.0, 0.5) |

In addition, the deep learning prediction model 10 may be trained by extracting a fully connected layer with a 4th residual block from a pretrained network by using the ImageNet 1k data set. At this time, the pretrained network may be implemented as ResNet-18. Here, ResNet-18 refers to a convolutional neural network composed of 18 layers. Building a different type of prediction model by taking a part from the pre-trained network is referred to as transfer learning. And the present invention can minimize the model memorizing training data by learning only a part of the model through the above-described method.

According to an embodiment, the deep learning prediction model 10 may be used in all networks with residual blocks, and accordingly, after performing the training about various networks such as ResNet, Wide ResNet, ResNext, and MobileNet V2, more accurate prediction can be derived by combining the predictions derived from the respective networks.

Further, the deep learning prediction model trained in the above-described manner has an improved recognition performance of image and classification capability of data compared to conventional deep learning prediction models.

According to an embodiment, the deep learning prediction model may use a function obtained by combining a Class Balanced (CB) loss function and a Generalized Cross Entropy (GCE) loss function as an objective function.

In this case, the CB loss function may be expressed as in [Equation 1] below.

[Equation 1]

$$CB(p,y) = \frac{1}{E_{n_y}} L(p,y) = \frac{1-\beta}{1-\beta^{n_y}} L(p,y)$$

Here, $E_n$ denotes a significant number indicating the expected volume of the sample, $\beta = (N-1)/N$, $N$ denotes the size of the data set, and $L(p,y)$ denotes the objective function (loss function).

If a model is trained using a dataset with an imbalanced number of data, such as medical data, the model may have a bias to classify an image into a class with a large number in the training data.

In other words, if there are many normal images in the training data, there is a high possibility of classifying patients with high severity as normal or low severity, so diagnosis of disease using this model causes serious errors.

Therefore, the deep learning prediction model 10 according to the present invention may perform an optimization process by using the CB loss function in order to have high accuracy regardless of data imbalance.

In this case, the GCE loss function may be expressed as in [Equation 2] below.

[Equation 2]

$$L_q(f(x),e_j) = \frac{(1-f_j(x)^q)}{q}$$

Here, $e_j$ is a one-hot vector whose j-th element is 1, and $f_j(x)$ denotes the j-th element of f(x).

The GCE loss is an objective function proposed to enable the robust training even when the labeling of an image is incorrect.

The data used for the training of the deep learning prediction model 10 may be ones whose labeling has been performed based on diagnosis results judged by clinicians after having observed images. In this case, since the data used for the training reflects individual bias, diagnosis of various levels may be made even with respect to the same image. If a model is trained using the labeling which has been performed under such subjective bias, it is hard to expect the accuracy of the trained model.

Therefore, the deep learning prediction model 10 according to the present invention may use the GCE loss as an objective function to increase the capability to make an objective diagnosis.

The value of the GCE loss function varies depending on the hyperparameter $q \in (0,1)$, and according to L'Hoptial's theorem, it is the same as the CE loss if $q \to 0$, and it is same as the Mean Absolute Error (MAE) loss if $q = 1$.

That is, the deep learning prediction model 10 according to an embodiment of the present invention compares a slit lamp microscopic examination result image or a retroillumination examination result image with reference data to classify the NO grade, NO grade, CO grade, and PSC grade of the subject, through which the degree of cataract progression can be estimated.

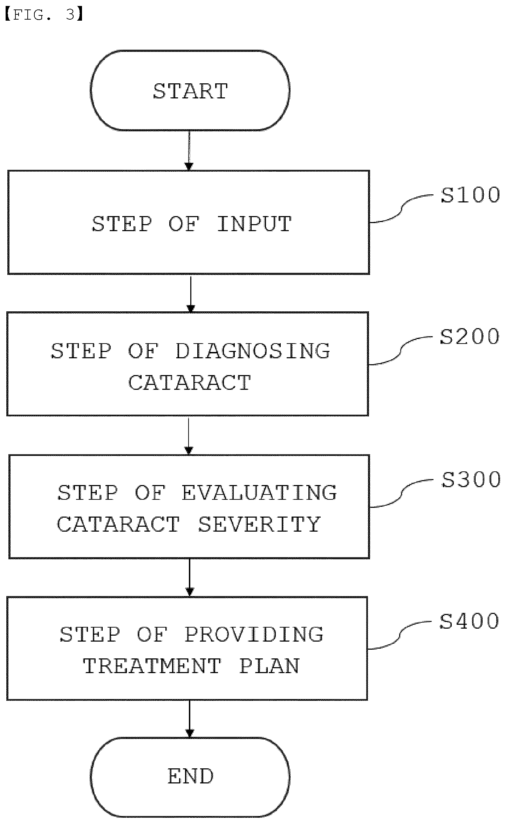

FIG. 3 is a flowchart of a method for diagnosis of cataract using deep learning according to the present invention.

Referring to FIG. 3, the method according to an embodiment of the present invention may include a step of input S100, a step of diagnosing cataract S200, a step of evaluating cataract severity S300, and a step of providing treatment plan S400.

In the step of input, an input unit 200 receives at least one of a slit lamp microscopic examination result image, a retroillumination examination result image, and a medical examination result of a subject.

The subject's medical examination result may include personal information such as age, name and the like of the subject, or body information such as height, weight, visual acuity information and the like.

According to an embodiment, in the step of input S100, a preprocessing unit 100 may first perform a step of preprocessing extracting only a region corresponding to the lens from the slit lamp microscopic examination result image by using Faster R-CNN.

This is for extracting and intensively analyzing only necessary information about, for example, the pupil region or a region including the lens of the subject because the training performance may be lowered due to the unnecessary region when information about, for example, parts other than the pupil region of the subject, which are not necessary for data analysis is included as shown in FIG. 1(a).

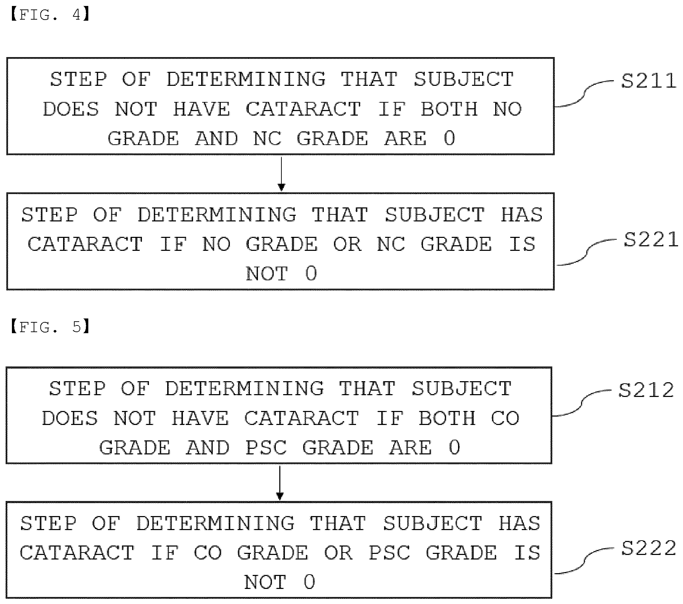

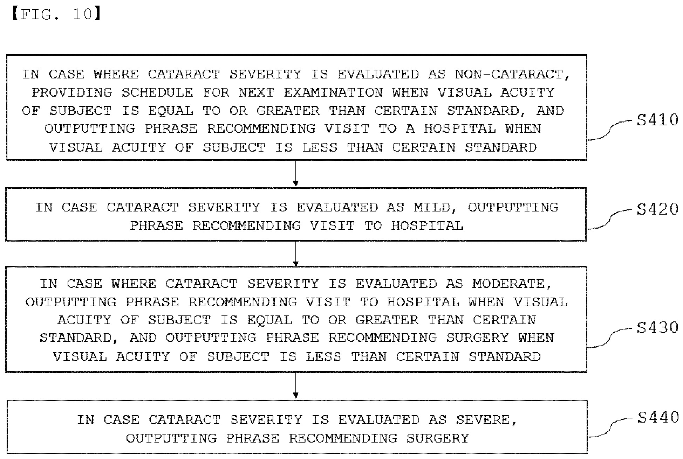

The step of diagnosing cataract S200 and the step of evaluating cataract severity S300 of the present invention will be described in detail below with reference to FIGS. 4 to 9, and the step of providing treatment plan S400 will be described in detail below with reference to FIG. 10.

FIGS. 4 and 5 show flowcharts of the step of diagnosing cataract S201 and the step of evaluating cataract severity S301 according to a first embodiment of the present invention.

Referring to FIGS. 4 and 5, the method for diagnosis of cataract according to the first embodiment of the present invention includes diagnosing cataract and evaluating the cataract severity by using the slit lamp microscopic examination result image.

In the step of diagnosing cataract S201, a cataract diagnosing unit 500 inputs the slit lamp microscopic examination result image into the deep learning prediction model 10 to estimate the degree of cataract progression in the lens nucleus, and determines whether the subject has cataract or not.

According to an embodiment, the step of diagnosing cataract S201 may further include the step of determining that the subject has no cataract when both the NO grade and the NO grade are 0 (S211), and the step of determining that the subject has cataract when the NO grade or NO grade is not 0 (S221).

That is, the cataract diagnosing unit 500 may input the slit lamp microscopic examination result image into the pretrained deep learning prediction model 10 to estimate the NO grade and NO grade of the inputted slit lamp microscopic examination result image. And the cataract diagnosing unit 500 may determine that the subject has no cataract when both the estimated NO grade and NO grade of the slit lamp microscopic examination result image are 0.

In the step of evaluating cataract severity S301, the severity evaluating unit 300 evaluates the cataract severity of the subject based on the degree of cataract progression in the lens nucleus. At this time, the cataract severity may be divided into non-cataract, mild, moderate, and severe cataract levels.

For example, the cataract severity may be classified as non-cataract when the degree of cataract progression (NO grade or NO grade) is grade 0; may be classified as mild when the degree of cataract progression (NO grade or NO grade) is grade 1 or 2; may be classified as moderate when the degree of cataract progression (NO grade or NO grade) is grade 3 or 4; and may be classified as severe when the degree of cataract progression (NO grade or NO grade) is grade 5 or 6.

That is, the cataract is classified as being more severe as the degree of cataract progression (i.e., the NO grade or NO grade) becomes higher.

According to an embodiment, the step of evaluating cataract severity S301 may further include the step of evaluating the degree of the cataract severity based on the greater value of the NO grade and the NO grade (S311), and the step of extracting the visual acuity of the subject from the medical examination result and determining whether or not the visual acuity is equal to or higher than a certain standard (S321).

For example, if the NO grade is grade 2 and the NO grade is grade 3, the cataract severity is evaluated based on grade 3, which is the greater of the two, so the cataract severity will be evaluated as moderate.

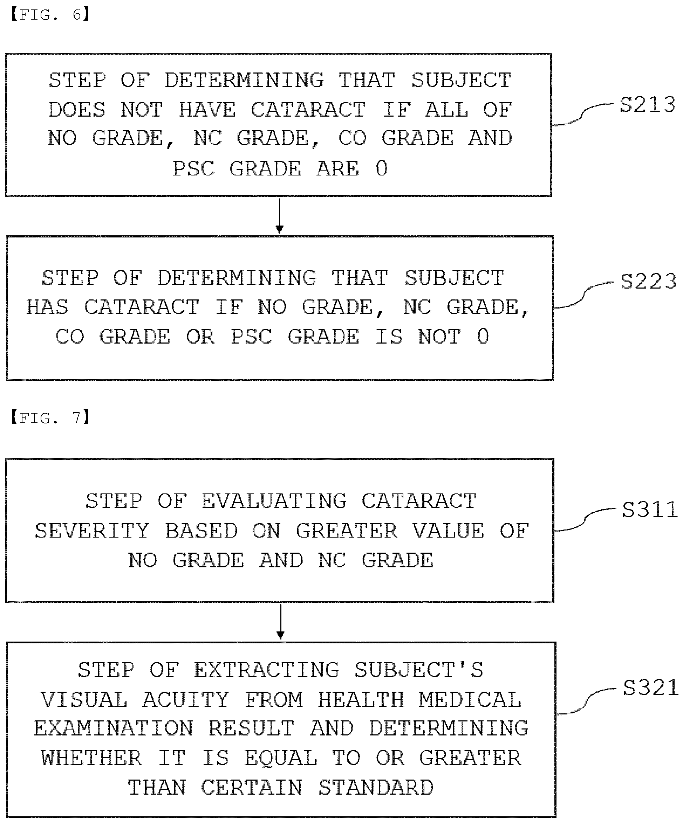

FIGS. 6 and 7 show flowcharts of the step of diagnosing cataract S202 and the the step of evaluating cataract severity S302 according to a second embodiment of the present invention.

Referring to FIGS. 6 and 7, the method for diagnosis of cataract according to the second embodiment of the present invention includes diagnosing cataract and evaluating the cataract severity by using the retroillumination examination result image.

In step of diagnosing cataract S202, the cataract diagnosing unit 500 inputs the retroillumination examination result image into the deep learning prediction model 10 to estimate the degrees of cataract progression in the cortex and posterior subcapsular of the lens, and determines whether the subject has cataract or not.

According to an embodiment, the step of diagnosing cataract S202 may further include the step of determining that the subject has no cataract when both the CO grade and the PSC grade are 0 (S212), and the step of determining that the subject has cataract when the CO grade or PSC grade is not 0 (S222).

That is, the cataract diagnosing unit 500 may input the retroillumination examination result image into the pretrained deep learning prediction model 10 to estimate the CO grade and PSC grade of the inputted retroillumination examination result image. And cataract diagnosing unit 500 may determine that the subject has no cataract when both the estimated CO grade and PSC grade of the retroillumination examination result image are 0.

In the step of evaluating cataract severity S302, the severity evaluating unit 300 evaluates the cataract severity of the subject based on the degree of cataract progression in the cortex and posterior subcapsular of the lens. At this time, the cataract severity may be divided into non-cataract, mild, moderate, and severe cataract levels.

For example, the cataract severity may be classified as non-cataract when the degree of cataract progression (CO grade or PSC grade) is grade 0; may be classified as mild when the degree of cataract progression (CO grade or PSC grade) is grade 1 or 2; may be classified as moderate when the degree of cataract progression (CO grade or PSC grade) is grade 3 or 4; and may be classified as severe when the degree of cataract progression (CO grade or PSC grade) is grade 5 or 6.

That is, the cataract is classified as being more severe as the degree of cataract progression (i.e., the CO grade or PSC grade) becomes higher.

According to an embodiment, the step of evaluating cataract severity S302 may further include the step of evaluating the degree of cataract severity based on the greater value of the CO grade and the PSC grade (S312), and the step of extracting the visual acuity of the subject from the medical examination result and determining whether or not the visual acuity is equal to or higher than a certain standard (S322).

For example, if the CO grade is grade 1 and the PSC grade is grade 5, the cataract severity is evaluated based on grade 5, which is the greater of the two, so the cataract severity will be evaluated as severe.

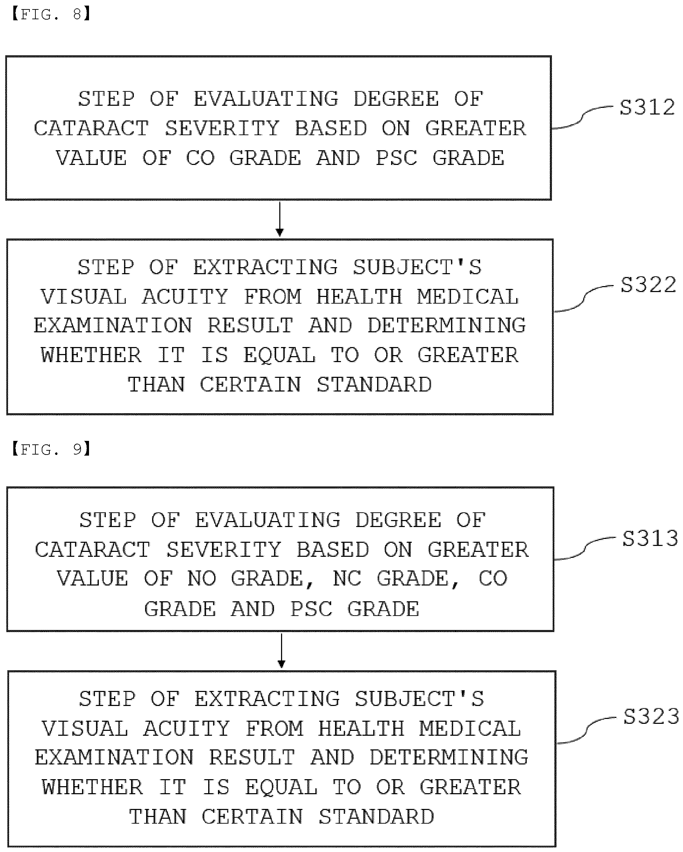

FIGS. 8 and 9 show flowcharts of the step of diagnosing cataract S203 and the step of evaluating cataract severity S303 according to a third embodiment of the present invention.

Referring to FIGS. 8 and 9, the method for diagnosis of cataract according to the third embodiment of the present invention includes diagnosing cataract and evaluating the cataract severity by using both the slit lamp microscopic examination result image and the retroillumination examination result image.

In the step of diagnosing cataract S200, the cataract diagnosing unit 500 inputs the slit lamp microscopic examination result image and the retroillumination examination result image into the deep learning prediction model 10 to estimate the degrees of the cataract progression in the nucleus, cortex and posterior subcapsular of the lens, and determines whether the subject has cataract or not.

According to an embodiment, the step of diagnosing cataract S200 may further include the step of determining that the subject has no cataract when all of the NO grade, the NC grade, the CO grade and the PSC grade are 0 (S213), and the step of determining that the subject has cataract when the NO grade, the NC grade, the CO grade or PSC grade is not 0 (S223).

That is, the cataract diagnosing unit 500 may input the slit lamp microscopic examination result image and the retroillumination examination result image into the pretrained deep learning prediction model 10 to estimate the NO grade, the NO grade, the CO grade and PSC grade of the inputted slit lamp microscopic examination result image and retroillumination examination result image, And cataract diagnosing unit 500 may determine that the subject has no cataract when all of the estimated NO grade, NC grade, CO grade and PSC grade of the slit lamp microscopic examination result image and the retroillumination examination result image are 0.

In the step of diagnosing cataract S303, the severity evaluating unit 300 evaluates the cataract severity of the subject based on the degree of cataract progression in the nucleus, cortex and posterior subcapsular of the lens. At this time, the cataract severity may be divided into non-cataract, mild, moderate, and severe cataract levels.

For example, the cataract severity may be classified as non-cataract when the degree of cataract progression (NO grade, NC grade, CO grade or PSC grade) is grade 0; may be classified as mild when the degree of cataract progression (NO grade, NC grade, CO grade or PSC grade) is grade 1 or 2; may be classified as moderate when the degree of cataract progression (NO grade, NC grade, CO grade or PSC grade) is grade 3 or 4; and may be classified as severe when the degree of cataract progression (NO grade, NC grade, CO grade or PSC grade) is grade 5 or 6.

That is, the cataract is classified as being more severe as the degree of cataract progression (i.e., the NO grade, the NO grade, the CO grade or PSC grade) becomes higher.

According to an embodiment, the step of evaluating cataract severity S300 may further include the step of evaluating the degree of the cataract severity based on the greater value of the NO grade, the NC grade, the CO grade and the PSC grade (S313), and the step of extracting the visual acuity of the subject from the medical examination result and determining whether or not the visual acuity is equal to or higher than a certain standard (S323).

For example, if the NO grade is grade 1, the NC grade is grade 2, the CO grade is grade 2 and the PSC grade is grade 1, the cataract severity is evaluated based on grade 2, which is the greatest of the four, so the cataract severity will be evaluated as mild.

According to an embodiment, evaluating the cataract severity based on the NO grade, NC grade, CO grade, and PSC grade may mean that the degree of the cataract severity is evaluated for each of the nucleus, cortex, and posterior subcapsular of the lens based on the NO grade, NC grade, CO grade, and PSC grade. In this case, according to an embodiment, the degree of the severity may be reevaluated based on the number of mild, moderate, or severe cases.

For example, if the NO grade is grade 3, the NC grade is grade 5, the CO grade is grade 2, and the PSC grade is grade 5, then the degree of cataract progression in the lens nucleus may be evaluated as severe (because the greater of the NO or NO grade is grade 5), the degree of cataract progression in the lens cortex may be evaluated as mild (because the CO grade is grade 2), and the degree of cataract progression in the posterior subcapsular of the lens may be evaluated as severe (because the PSC grade is grade 5) .

At this time, the degree of severe cataract progression may be further subdivided based on the fact that 2 out of 3 degrees of cataract progression in the nucleus, cortex, and posterior subcapsular of the lens are evaluated as severe (e.g., may be subdivided into 0 severe, 1 severe, or 2 severes).

FIG. 10 is a flowchart of the step of providing treatment plan S400 according to an embodiment of the present invention.

Referring to FIG. 10, in the step of providing treatment plan S400 according to an embodiment of the present invention, a treatment stage determining unit 400 determines and provides a necessary treatment stage of cataract for the subject by using the cataract severity or the medical examination result.

According to an embodiment, the step of providing treatment plan S400 may further include in a case where the cataract severity is evaluated as non-cataract, a step of providing a schedule for a next examination when the visual acuity of the subject is equal to or greater than a certain standard, and outputting a phrase recommending a visit to a hospital when the visual acuity of the subject is less than a certain standard (S410), a step of outputting a phrase recommending a visit to a hospital when the cataract severity is evaluated as mild (S420), in a case where the cataract severity is evaluated as moderate, a step of outputting a phrase recommending a visit to a hospital when the visual acuity of the subject is equal to or greater than a certain standard, and outputting a phrase recommending surgery when the visual acuity of the subject is less than a certain standard (S430), and a step of outputting a phrase recommending surgery when the cataract severity is evaluated as severe (S440).

In other words, even if it is determined that there is no cataract, it may be determined and provided that the next examination schedule will be provided to the subject, or that a recommendation will be made for the subject to visit a hospital and receive treatment from a clinician to check for other causes of decreased visual acuity, based on whether the subject's visual acuity is problematic. And when the cataract severity is moderate, it may be determined and provided that the phrase recommending surgery will be outputted, or that the phrase recommending that the subject visits a hospital and receives treatment from a clinician, based on whether the subject's visual acuity is problematic.

Furthermore, the present invention may provide a computer-readable recording medium for implementing the diagnosis method of FIGS. 3 to 10 as a program. In other words, the above method can be written as a program that can be executed on a computer, and can be implemented in a general-purpose digital computer which operates the program using a computer-readable medium. In addition, the structure of data used in the above method may be recorded on a computer readable medium through various means.

It should not be understood that a recording medium for recording an executable computer program or code for performing various methods of the present invention includes temporary targets such as carrier waves or signals. The computer-readable medium may include storage media, such as magnetic storage media (e.g., a ROM, a floppy disk, a hard disk and the like) and optical reading media (e.g., CD-ROM, DVD and the like).

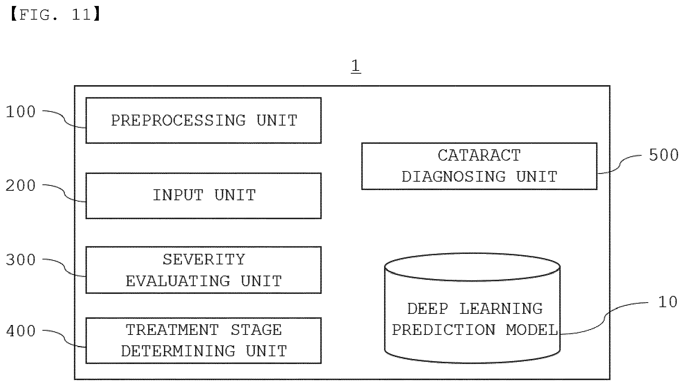

FIG. 11 shows a block diagram of a system for diagnosis of cataract using deep learning 1 according to an embodiment of the present invention.

Referring to FIG. 11, the system for diagnosis of cataract using deep learning 1 according to an embodiment of the present invention may include the input unit 200 obtaining a slit lamp microscopic examination result image, a retroillumination examination result image, or a medical examination result of a subject, the deep learning prediction model 10 pretrained based on the slit lamp microscopic examination result image or the retroillumination examination result image; the cataract diagnosing unit 500 that estimates the degree of cataract progression in the nucleus, cortex, or posterior subcapsular region of the lens using the deep learning prediction model 10 and determines whether the subject has cataract based on the degree of cataract progression, the severity evaluating unit 300 for evaluating the cataract severity based on the degree of the cataract progress; and the treatment stage determining unit 400 that determines and provides a necessary treatment stage of cataract for the subject using the cataract severity or the medical examination result.

The system for diagnosis of cataract using deep learning 1 according to an embodiment of the present invention may further include a preprocessing unit 100 extracting only a region corresponding to the lens from the slit lamp microscopic examination result image or the retroillumination examination result image by using Faster R-CNN.

The preprocessing unit 100 may increase the number of data used for the training of the deep learning prediction model 10 by modifying the slit lamp microscopic examination result image and the retroillumination examination result image.

According to an embodiment, the deep learning prediction model 10 may be trained by extracting a fully connected layer with a 4th residual block from a pretrained network by using the ImageNet 1k data set.

At this time, the pretrained network may be implemented as ResNet-18. Here, ResNet-18 refers to a convolutional neural network composed of 18 layers.

According to an embodiment, the deep learning prediction model 10 may use, as an objective function, a function obtained by combining a Class Balanced (CB) loss function and a Generalized Cross Entropy (GCE) loss function.

According to an embodiment, the deep learning prediction model 10 may be used in all networks with residual blocks, and accordingly, after performing the training about various networks such as ResNet, Wide ResNet, ResNext, and MobileNet V2, more accurate prediction can be derived by combining the predictions derived from the respective networks.

In relation to the system according to an embodiment of the present invention, the contents of the above-described methods may be applied. Therefore, descriptions of the same contents as those of the above-described method in relation to the system will be omitted.

As results of experiments which have been performed to evaluate the accuracy of the method and system for diagnosis according to an embodiment of the present invention, the experimental results shown in [Table 2] below could be obtained.

[Table 2]

| Standard for degree of cataract progression | Accuracy |
|---|---|
| NO grade | 93.62% |
| NC grade | 92.57% |
| CO grade | 90.87% |
| PSC grade | 90.28% |

That is, according to [Table 1], it can be seen that the method and system for diagnosis according to an embodiment of the present invention have classification accuracies of 93.62% and 92.57% for the NO grade and NC grade, respectively, representing the degrees of cataract progression in the lens nucleus. And, it can be seen that the method and system for diagnosis according to an embodiment of the present invention have classification accuracy of 90.87% for the CO grade representing the degree of cataract progression in the lens cortex, and have classification accuracy of 90.28% for the PSC grade representing the degree of cataract progression in the posterior subcapsular of the lens. These are higher than the conventional classification accuracies of (NO grade) 61.51%, (NC grade) 70.94%, (CO grade) 58.75%, and (PSC grade) 64.89%. That is, the accuracy of cataract diagnosis can be improved by the diagnosis method and diagnosis system according to an embodiment of the present invention. In the above experiments, the deep learning prediction models were classified into Region Detection Network (RDN) and Classification Network (CN), and were implemented through training the RDN first (Step 1), fixing the trained RDN and then training the CN (Step 2).

In Step 1 which is a process of extracting the position corresponding to the lens using the ImageNet-1K data set, the already learned RDN was trained using a standard smoothing loss function as shown in [Equation 3] below.

At this time, an optimizer with a learning rate of 0.005, momentum of 0.9, and weight decay coefficient of 0.0005 was used, and the network was trained for 10 epochs of mini-batch size 8, and when a cropped image was given to the RDN, the size was adjusted to 224x224 and normalized from 0 to 1.

[Equation 3]

$$smooth_{L_i}(x) = \int \begin{matrix} 0.5x^2 & if, |x| < 1 \\ |x| - 0.5 & otherwise \end{matrix}$$

In Step 2 which is a process of estimating two types of grades for each image type, CN was trained with a method of minimizing the CB loss function with the GCE loss function as the objective function.

In this regard, the Adam optimization program with basic hyperparameters was used, and the weight decay coefficient was set to 0.0005. In addition, the learning rate was initially set to 0.001 for the fully connected layer, and set to 0.0001 for the last remaining block. For the convergence of the next training, the learning rate was reduced by 0.95 times every 10 epochs, and the network was trained for 200 epochs with a mini-batch size of 32.

The aforementioned description of the present invention is just an example, and a person having ordinary skill in the art to which the present invention pertains may understand that it can be easily modified into other specific configuration without changing the technical idea or essential features of the present invention. Accordingly, it should be understood that the embodiments described above are illustrative and not restrictive in every respect. For example, the respective components described as a singular form may be implemented in a distributed form, and likewise the respective components described as a distributed form may be implemented in a combined form.

The scope of the disclosure is defined by the following claims rather than the detailed description, and all changed or modified forms derived from the meaning and scope of the claims and equivalents thereto should be interpreted as being included in the scope of the disclosure.

**Claims**

1. A method for diagnosis of cataract using deep learning, the method comprising:

   a step of input in which an input unit receives a slit lamp microscopic examination result image and a medical examination result of a subject;
   a step of diagnosing cataract in which a cataract diagnosing unit inputs the slit lamp microscopic examination result image into a deep learning prediction model to estimate the degree of cataract progression in a lens nucleus, and determines whether the subject has cataract;
   a step of evaluating cataract severity in which a severity evaluating unit evaluates the cataract severity of the subject based on the degree of cataract progression in the lens nucleus; and
   a step of providing treatment plan in which a treatment stage determining unit determines and provides a necessary treatment stage of cataract for the subject using the cataract severity or the medical examination result.

2. The method for diagnosis of cataract using deep learning of claim 1, further comprising:
   before the step of input, a step of preprocessing in which a preprocessing unit extracts only a region corresponding to the lens from the slit lamp microscopic examination result image using Faster R-CNN.

3. The method for diagnosis of cataract using deep learning of claim 1, wherein the degree of cataract progression in the lens nucleus is determined by using an NO grade in which grades are classified based on the degree of opacity of the lens nucleus and an NO grade in which grades are classified based on the degree of browning of the lens nucleus.

4. The method for diagnosis of cataract using deep learning of claim 3, wherein the step of diagnosing cataract further includes a step of determining that the subject has no cataract when both the NO grade and the NO grade are 0, and a step of determining that the subject has cataract when the NO grade or NO grade is not 0.

5. The method for diagnosis of cataract using deep learning of claim 4, wherein the cataract severity is divided into non-cataract, mild, moderate, and severe cataract levels, and
   wherein the step of evaluating cataract severity further includes a step of evaluating the degree of cataract severity based on the greater value of the NO grade and the NO grade, and a step of extracting the visual acuity of the subject from the medical examination result and determining whether or not the visual acuity is equal to or greater than a certain standard.

6. The method for diagnosis of cataract using deep learning of claim 5, wherein the step of providing treatment plan further includes:

   in a case where the cataract severity is evaluated as non-cataract, a step of providing a schedule for a next examination when the visual acuity of the subject is equal to or greater than a certain standard, and outputting a phrase recommending a visit to a hospital when the visual acuity of the subject is less than a certain standard;
   in a case where the cataract severity is evaluated as mild, a step of outputting a phrase recommending a visit to a hospital;
   in a case where the cataract severity is evaluated as moderate, a step of outputting a phrase recommending a visit to a hospital when the visual acuity of the subject is equal to or greater than a certain standard, and outputting a phrase recommending surgery when the visual acuity of the subject is less than a certain standard; and
   in a case where the cataract severity is evaluated as severe, a step of outputting a phrase recommending surgery.

7. A method for diagnosis of cataract using deep learning, the method comprising:

   a step of input in which an input unit receives a retroillumination examination result image and a medical examination result of a subject;

a step of diagnosing cataract in which a cataract diagnosing unit inputs the retroillumination examination result image into a deep learning prediction model to estimate the degrees of cataract progression in a cortex and a posterior subcapsular of a lens, and determines whether the subject has cataract;

a step of evaluating cataract severity in which a severity evaluating unit evaluates the cataract severity of the subject based on the degrees of cataract progression in the cortex and the posterior subcapsular of the lens; and

a step of providing treatment plan in which a treatment stage determining unit determines and provides a necessary treatment stage of cataract for the subject using the cataract severity or the medical examination result.

8. The method for diagnosis of cataract using deep learning of claim 7, further comprising:
before the step of input, a step of preprocessing in which a preprocessing unit extracts only a region corresponding to the lens from the retroillumination examination result image using Faster R-CNN.

9. The method for diagnosis of cataract using deep learning of claim 7, wherein the degree of cataract progression in the cortex of lens is determined by using a CO grade in which grades are classified based on whether opaque opacity exists in the lens cortex portion of the retroillumination examination result image and on an area occupied by the opaque opacity in the entire lens, and

wherein the degree of cataract progression in the posterior subcapsular of the lens is determined by using a PSC grade in which grades are classified based on whether opaque opacity exists in the posterior part of the lens of the retroillumination examination result image and on an area occupied by the opaque opacity in the entire lens.

10. The method for diagnosis of cataract using deep learning of claim 9, wherein the step of diagnosing cataract further includes a step of determining that the subject has no cataract when both the CO grade and the PSC grade are 0, and a step of determining that the subject has cataract when the CO grade or PSC grade is not 0.

11. The method for diagnosis of cataract using deep learning of claim 10, wherein the cataract severity is divided into non-cataract, mild, moderate, and severe cataract levels, and

wherein the step of evaluating cataract severity further includes a step of evaluating the degree of cataract severity based on the greater value of the CO grade and the PSC grade, and a step of extracting the visual acuity of the subject from the medical examination result and determining whether or not the visual acuity is equal to or greater than a certain standard.

12. The method for diagnosis of cataract using deep learning of claim 11, wherein the step of providing treatment plan further includes:

in a case where the cataract severity is evaluated as non-cataract, a step of providing a schedule for a next examination when the visual acuity of the subject is equal to or greater than a certain standard, and outputting a phrase recommending a visit to a hospital when the visual acuity of the subject is less than a certain standard;

in a case where the cataract severity is evaluated as mild, a step of outputting a phrase recommending a visit to a hospital;

in a case where the cataract severity is evaluated as moderate, a step of outputting a phrase recommending a visit to a hospital when the visual acuity of the subject is equal to or greater than a certain standard, and outputting a phrase recommending surgery when the visual acuity of the subject is less than a certain standard; and

in a case where the cataract severity is evaluated as severe, a step of outputting a phrase recommending surgery.

13. A method for diagnosis of cataract using deep learning, the method comprising:

a step of input in which an input unit receives a slit lamp microscopic examination result image, a retroillumination examination result image and a medical examination result of a subject;

a step of diagnosing cataract in which a cataract diagnosing unit inputs the slit lamp microscopic examination result image, the retroillumination examination result image into a deep learning prediction model to estimate the degrees of cataract progression in a nucleus, a cortex and a posterior subcapsular of a lens, and determines whether the subject has cataract;

a step of evaluating cataract severity in which a severity evaluating unit evaluates the cataract severity of the subject based on the degrees of cataract progression in the nucleus, the cortex and the posterior subcapsular of the lens; and

a step of providing treatment plan in which a treatment stage determining unit determines and provides a necessary treatment stage of cataract for the subject using the cataract severity or the medical examination result.

14. The method for diagnosis of cataract using deep learning of claim 13, further comprising:
before the step of input, a step of preprocessing in which a preprocessing unit extracts only a region corresponding to the lens from the slit lamp microscopic examination result image or the retroillumination examination result image using Faster R-CNN.

15. The method for diagnosis of cataract using deep learning of claim 13, wherein the degree of cataract progression in the lens nucleus is determined by using an NO grade in which grades are classified based on the degree of opacity of the lens nucleus and an NC grade in which grades are classified based on the degree of browning of the lens nucleus,

wherein the degree of cataract progression in the cortex of lens is determined by using a CO grade in which grades are classified based on whether opaque opacity exists in the lens cortex portion of the retroillumination examination result image and on an area occupied by the opaque opacity in the entire lens, and
wherein the degree of cataract progression in the posterior subcapsular of the lens is determined by using a PSC grade in which grades are classified based on whether opaque opacity exists in the posterior part of the lens of the retroillumination examination result image and on an area occupied by the opaque opacity in the entire lens.

16. The method for diagnosis of cataract using deep learning of claim 15, wherein the step of diagnosing cataract further includes a step of determining that the subject has no cataract when all of the NO grade, the NO grade, the CO grade and the PSC grade are 0; and a step of determining that the subject has cataract when the NO grade, the NC grade, the CO grade or PSC grade is not 0.

17. The method for diagnosis of cataract using deep learning of claim 16, wherein the cataract severity is divided into non-cataract, mild, moderate, and severe cataract levels, and
wherein the step of evaluating cataract severity further includes a step of evaluating the degree of cataract severity based on the greater value of the NO grade, the NC grade, the CO grade and the PSC grade, and a step of extracting the visual acuity of the subject from the medical examination result and determining whether or not the visual acuity is equal to or greater than a certain standard.

18. The method for diagnosis of cataract using deep learning of claim 17, wherein the step of providing treatment plan further includes:

in a case where the cataract severity is evaluated as non-cataract, a step of providing a schedule for a next examination when the visual acuity of the subject is equal to or greater than a certain standard, and outputting a phrase recommending a visit to a hospital when the visual acuity of the subject is less than a certain standard;
in a case where the cataract severity is evaluated as mild, a step of outputting a phrase recommending a visit to a hospital;
in a case where the cataract severity is evaluated as moderate, a step of outputting a phrase recommending a visit to a hospital when the visual acuity of the subject is equal to or greater than a certain standard, and outputting a phrase recommending surgery when the visual acuity of the subject is less than a certain standard; and
in a case where the cataract severity is evaluated as severe, a step of outputting a phrase recommending surgery.

19. A computer-readable recording medium on which a program for implementing a method of any one of claims 1 to 18 is recorded.

20. A system for diagnosis of cataract using deep learning, the system comprising:

an input unit obtaining a slit lamp microscopic examination result image, a retroillumination examination result image, or a medical examination result of a subject;
a deep learning prediction model pretrained based on the slit lamp microscopic examination result image or the retroillumination examination result image;
a cataract diagnosing unit which estimates the degree of cataract progression in the nucleus, cortex, or posterior subcapsular of a lens using the deep learning prediction model and determines whether the subject has cataract based on the degree of cataract progression;
a severity evaluating unit which evaluates cataract severity based on the degree of the cataract progression; and
a treatment stage determining unit which determines and provides a necessary treatment stage of cataract for the subject using the cataract severity or the medical examination result.

**21.** The system for diagnosis of cataract using deep learning of claim 20, further comprising a preprocessing unit which extracts only a region corresponding to the lens from the slit lamp microscopic examination result image or the retroillumination examination result image using Faster R-CNN.

**22.** The system for diagnosis of cataract using deep learning of claim 21, wherein the preprocessing unit increases the number of data used for the training of the deep learning prediction model by modifying the slit lamp microscopic examination result image and the retroillumination examination result image.

**23.** The system for diagnosis of cataract using deep learning of claim 20, wherein the deep learning prediction model is trained by extracting a fully connected layer with a fourth residual block from a pretrained network by using an ImageNet 1k data set.

**24.** The system for diagnosis of cataract using deep learning of claim 20, wherein the deep learning prediction model uses, as an objective function, a function obtained by combining a Class Balanced (CB) loss function and a Generalized Cross Entropy (GCE) loss function.

**25.** The system for diagnosis of cataract using deep learning of claim 20, wherein the deep learning prediction model is used in a network with a residual block, and is implemented by being trained on at least one network among ResNet, Wide ResNet, ResNext, and MobileNet V2, and then combining the predictions derived from the respective the networks.

【FIG. 1】

(a)

(b)

【FIG. 2】

【FIG. 3】

```
          ┌─────────────┐
          │    START    │
          └──────┬──────┘
                 │
                 ▼
    ┌─────────────────────────┐
    │     STEP OF INPUT       │──── S100
    └────────────┬────────────┘
                 │
                 ▼
    ┌─────────────────────────┐
    │   STEP OF DIAGNOSING     │──── S200
    │        CATARACT          │
    └────────────┬────────────┘
                 │
                 ▼
    ┌─────────────────────────┐
    │   STEP OF EVALUATING     │──── S300
    │    CATARACT SEVERITY     │
    └────────────┬────────────┘
                 │
                 ▼
    ┌─────────────────────────┐
    │   STEP OF PROVIDING      │──── S400
    │     TREATMENT PLAN       │
    └────────────┬────────────┘
                 │
                 ▼
          ┌─────────────┐
          │     END     │
          └─────────────┘
```

【FIG. 4】

```
┌─────────────────────────────────────┐
│    STEP OF DETERMINING THAT SUBJECT  │ ─── S211
│  DOES NOT HAVE CATARACT IF BOTH NO   │
│      GRADE AND NC GRADE ARE 0        │
└─────────────────────────────────────┘
                    │
                    ▼
┌─────────────────────────────────────┐
│  STEP OF DETERMINING THAT SUBJECT HAS│
│  CATARACT IF NO GRADE OR NC GRADE IS │ ─── S221
│               NOT 0                  │
└─────────────────────────────────────┘
```

【FIG. 5】

```
┌─────────────────────────────────────┐
│    STEP OF DETERMINING THAT SUBJECT  │ ─── S212
│  DOES NOT HAVE CATARACT IF BOTH CO   │
│      GRADE AND PSC GRADE ARE 0       │
└─────────────────────────────────────┘
                    │
                    ▼
┌─────────────────────────────────────┐
│  STEP OF DETERMINING THAT SUBJECT HAS│
│  CATARACT IF CO GRADE OR PSC GRADE IS│ ─── S222
│               NOT 0                  │
└─────────────────────────────────────┘
```

【FIG. 6】

| |
|---|
| STEP OF DETERMINING THAT SUBJECT DOES NOT HAVE CATARACT IF ALL OF NO GRADE, NC GRADE, CO GRADE AND PSC GRADE ARE 0 |

S213

↓

| |
|---|
| STEP OF DETERMINING THAT SUBJECT HAS CATARACT IF NO GRADE, NC GRADE, CO GRADE OR PSC GRADE IS NOT 0 |

S223

【FIG. 7】

| |
|---|
| STEP OF EVALUATING CATARACT SEVERITY BASED ON GREATER VALUE OF NO GRADE AND NC GRADE |

S311

↓

| |
|---|
| STEP OF EXTRACTING SUBJECT'S VISUAL ACUITY FROM HEALTH MEDICAL EXAMINATION RESULT AND DETERMINING WHETHER IT IS EQUAL TO OR GREATER THAN CERTAIN STANDARD |

S321

【FIG. 8】

```
STEP OF EVALUATING DEGREE OF
CATARACT SEVERITY BASED ON GREATER          S312
VALUE OF CO GRADE AND PSC GRADE
```

```
STEP OF EXTRACTING SUBJECT'S
VISUAL ACUITY FROM HEALTH MEDICAL
EXAMINATION RESULT AND DETERMINING          S322
WHETHER IT IS EQUAL TO OR GREATER
THAN CERTAIN STANDARD
```

【FIG. 9】

```
STEP OF EVALUATING DEGREE OF
CATARACT SEVERITY BASED ON GREATER
VALUE OF NO GRADE, NC GRADE, CO             S313
GRADE AND PSC GRADE
```

```
STEP OF EXTRACTING SUBJECT'S
VISUAL ACUITY FROM HEALTH MEDICAL
EXAMINATION RESULT AND DETERMINING          S323
WHETHER IT IS EQUAL TO OR GREATER
THAN CERTAIN STANDARD
```

【FIG. 10】

IN CASE WHERE CATARACT SEVERITY IS EVALUATED AS NON-CATARACT, PROVIDING SCHEDULE FOR NEXT EXAMINATION WHEN VISUAL ACUITY OF SUBJECT IS EQUAL TO OR GREATER THAN CERTAIN STANDARD, AND OUTPUTTING PHRASE RECOMMENDING VISIT TO A HOSPITAL WHEN VISUAL ACUITY OF SUBJECT IS LESS THAN CERTAIN STANDARD — S410

IN CASE CATARACT SEVERITY IS EVALUATED AS MILD, OUTPUTTING PHRASE RECOMMENDING VISIT TO HOSPITAL — S420

IN CASE WHERE CATARACT SEVERITY IS EVALUATED AS MODERATE, OUTPUTTING PHRASE RECOMMENDING VISIT TO HOSPITAL WHEN VISUAL ACUITY OF SUBJECT IS EQUAL TO OR GREATER THAN CERTAIN STANDARD, AND OUTPUTTING PHRASE RECOMMENDING SURGERY WHEN VISUAL ACUITY OF SUBJECT IS LESS THAN CERTAIN STANDARD — S430

IN CASE CATARACT SEVERITY IS EVALUATED AS SEVERE, OUTPUTTING PHRASE RECOMMENDING SURGERY — S440

【FIG. 11】

<div align="center">**INTERNATIONAL SEARCH REPORT**</div>

| International application No. |
|---|
| **PCT/KR2022/005134** |

**A.  CLASSIFICATION OF SUBJECT MATTER**

**A61B 3/117**(2006.01)i; **A61B 3/00**(2006.01)i; **G16H 50/20**(2018.01)i; **G16H 50/30**(2018.01)i; **G16H 30/40**(2018.01)i; **A61B 3/135**(2006.01)i; **G06N 3/08**(2006.01)i; **G06N 3/04**(2006.01)i

According to International Patent Classification (IPC) or to both national classification and IPC

**B.  FIELDS SEARCHED**

Minimum documentation searched (classification system followed by classification symbols)

A61B 3/117(2006.01); A61B 3/10(2006.01); A61B 3/14(2006.01); A61B 5/00(2006.01); A61K 31/195(2006.01); A61K 31/4015(2006.01); G06N 20/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 딥러닝(deep learning), 백내장(cataract), 세극등 현미경(slit lamp), 역반사조명 (retroillumination), 심각성(severity)

**C.  DOCUMENTS CONSIDERED TO BE RELEVANT**

| Category* | Citation of document, with indication, where appropriate, of the relevant passages | Relevant to claim No. |
|---|---|---|
| X | JP 2015-216939 A (MIKI HOLDINGS:KK et al.) 07 December 2015 (2015-12-07) See claims 3, 4 and 7; paragraphs [0024], [0027]-[0030], [0033], [0037] and [0051]-[0054]; tables 2-4; and figure 1. | 1,3-5,19,20 |
| Y | | 2,6-18,21-25 |
| Y | KR 10-2015-0130361 A (UNIVERSITY OF MASSACHUSETTS) 23 November 2015 (2015-11-23) See paragraph [0142]. | 7-18 |
| Y | KR 10-2021-0012097 A (INDUSTRY-ACADEMIC COOPERATION FOUNDATION, DANKOOK UNIVERSITY) 03 February 2021 (2021-02-03) See claim 1. | 2,8,14,21,22 |
| Y | KR 10-2208508 B1 (CMLAB CO., LTD.) 27 January 2021 (2021-01-27) See claims 1 and 2; and paragraphs [0066]-[0068]. | 6,12,18 |

| ✓ Further documents are listed in the continuation of Box C. | ✓ See patent family annex. |
|---|---|

\*    Special categories of cited documents:
"A"   document defining the general state of the art which is not considered to be of particular relevance
"D"   document cited by the applicant in the international application
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

| Date of the actual completion of the international search | Date of mailing of the international search report |
|---|---|
| **18 July 2022** | **21 July 2022** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2022/005134** |

| C. | DOCUMENTS CONSIDERED TO BE RELEVANT | |
| --- | --- | --- |
| Category* | Citation of document, with indication, where appropriate, of the relevant passages | Relevant to claim No. |
| Y | KR 10-2021-0073388 A (SAMSUNG LIFE PUBLIC WELFARE FOUNDATION et al.) 18 June 2021 (2021-06-18)<br>    See claims 1 and 7; and paragraphs [0083] and [0094]-[0097]. | 23-25 |

Form PCT/ISA/210 (second sheet) (July 2019)

## INTERNATIONAL SEARCH REPORT
### Information on patent family members

| International application No. |
|---|
| **PCT/KR2022/005134** |

| Patent document cited in search report | | | Publication date (day/month/year) | Patent family member(s) | | | Publication date (day/month/year) |
|---|---|---|---|---|---|---|---|
| JP | 2015-216939 | A | 07 December 2015 | JP | 6361065 | B2 | 25 July 2018 |
| KR | 10-2015-0130361 | A | 23 November 2015 | AU | 2014-236582 | A1 | 05 November 2015 |
| | | | | AU | 2014-236582 | B2 | 29 March 2018 |
| | | | | BR | 112015023348 | A2 | 18 July 2017 |
| | | | | CA | 2904657 | A1 | 25 September 2014 |
| | | | | CA | 2904657 | C | 16 February 2021 |
| | | | | CL | 2015002689 | A1 | 05 February 2016 |
| | | | | CN | 105209033 | A | 30 December 2015 |
| | | | | CN | 105209033 | B | 18 September 2018 |
| | | | | CR | 20150537 | A | 27 November 2015 |
| | | | | EA | 029070 | B1 | 28 February 2018 |
| | | | | EA | 201591677 | A1 | 29 January 2016 |
| | | | | EP | 2968239 | A1 | 20 January 2016 |
| | | | | EP | 2968239 | B1 | 24 April 2019 |
| | | | | ES | 2727293 | T3 | 15 October 2019 |
| | | | | HK | 1218390 | A1 | 17 February 2017 |
| | | | | IL | 241306 | A | 30 November 2015 |
| | | | | IL | 241306 | D0 | 30 November 2015 |
| | | | | JP | 2016-517428 | A | 16 June 2016 |
| | | | | JP | 6397477 | B2 | 26 September 2018 |
| | | | | KR | 10-2198622 | B1 | 05 January 2021 |
| | | | | MX | 2015013034 | A | 05 July 2016 |
| | | | | MX | 366115 | B | 27 June 2019 |
| | | | | NZ | 713007 | A | 27 November 2020 |
| | | | | PE | 17472015 | A1 | 18 December 2015 |
| | | | | PE | 20151747 | A1 | 18 December 2015 |
| | | | | SA | 515361106 | B1 | 25 September 2018 |
| | | | | SG | 11201507209 | A | 29 October 2015 |
| | | | | US | 10413529 | B2 | 17 September 2019 |
| | | | | US | 2016-0000707 | A1 | 07 January 2016 |
| | | | | US | 2016-0074370 | A1 | 17 March 2016 |
| | | | | US | 2017-0231905 | A1 | 17 August 2017 |
| | | | | US | 9675589 | B2 | 13 June 2017 |
| | | | | US | 9789091 | B2 | 17 October 2017 |
| | | | | WO | 2014-152818 | A1 | 25 September 2014 |
| | | | | ZA | 201506987 | B | 21 December 2016 |
| KR | 10-2021-0012097 | A | 03 February 2021 | KR | 10-2313143 | B1 | 18 October 2021 |
| KR | 10-2208508 | B1 | 27 January 2021 | KR | 10-2019-0043494 | A | 26 April 2019 |
| KR | 10-2021-0073388 | A | 18 June 2021 | KR | 10-2329313 | B1 | 19 November 2021 |
| | | | | WO | 2021-118255 | A2 | 17 June 2021 |
| | | | | WO | 2021-118255 | A3 | 05 August 2021 |

Form PCT/ISA/210 (patent family annex) (July 2019)